# FASCICULE DE BREVET EUROPEEN

(11) **EP 0 883 595 B1**
(45) Date de publication et mention de la délivrance du brevet: **07.03.2001**
(21) Numéro de dépôt: 96941077.8
(22) Date de dépôt: 29.11.1996
(51) Int. Cl.: C07C 209/36, C07C 211/51

(54) **PROCEDE D'HYDROGENATION DE COMPOSES NITRES AROMATIQUES**
VERFAHREN ZUR HYDRIERUNG AROMATISCHER NITROVERBINDUNGEN
METHOD FOR HYDROGENATING AROMATIC NITRO COMPOUNDS

(30) Priorité: 01.12.1995 FR 9514203
(43) Date de publication de la demande: 16.12.1998
(73) Titulaire: RHODIA CHIMIE, 92408 Courbevoie Cédex (FR)
(72) Inventeur: CHAMBOST, Bernard, F-69006 Lyon (FR); MARION, Philippe, F-69390 Vernaison (FR); MATHIEU, Corinne, F-69360 Ternay (FR)
(74) Mandataire: Wattremez, Catherine
(86) Numéro de dépôt international: FR9601893
(87) Numéro de publication internationale: WO9720804

(56) Documents cités:
- EP-A- 0 696 573
- FR-A- 2 126 832
- GB-A- 1 452 466
- CHEMICAL ABSTRACTS, vol. 92, no. 14, 7 Avril 1980 Columbus, Ohio, US; abstract no. 112780k, page 106; colonne 2; XP002009186 & CS 179 659 A (PASEK JOSEF ET AL.)
- CHEMICAL ABSTRACTS, vol. 100, no. 16, 16 Avril 1984 Columbus, Ohio, US; abstract no. 123155v, GRAMATIKOV, K. ET AL.: "Adiabatic process of catalytic reduction of nitrobenzene to aniline" page 108; colonne 2; XP002009187 & IZV. KHIM., vol. 16, no. 1-2, 1983, pages 38-43,
- CHEMICAL ABSTRACTS, vol. 117, no. 20, 16 Novembre 1992 Columbus, Ohio, US; abstract no. 194013j, page 120; colonne 2; XP002009188 & CS 263 430 A (ROZINEK, RADOVAN ET AL.)

## Description

La présente invention a trait à l'hydrogénation de composés nitrés aromatiques en mettant en oeuvre un catalyseur en lit fixe.

Parmi les procédés de ce type, on peut dénombrer deux catégories, la première mettant en oeuvre un catalyseur sous forme de suspension agitée, la seconde mettant en oeuvre un catalyseur en lit fixe.

Les procédés mettant en jeu des catalyseurs sous forme de suspension agitée ont fait l'objet de développements industriels importants. En effet, malgré un investissement élevé, ils présentent l'avantage de contrôler efficacement l'exothermie de la réaction d'hydrogénation, qui est importante pour ce type de composés. Par ailleurs, l'hydrogénation de composés nitrés sur catalyseur en suspension agitée présente de bonnes performances, en terme de sélectivité en composé souhaité, de conversion du produit nitré et en productivité. Enfin, le remplacement du catalyseur est relativement facile pour ces procédés, ce qui contribue à la conservation de leurs bonnes performances.

L'autre catégorie consistant à employer des catalyseurs en lit fixe pour l'hydrogénation de composés nitrés aromatiques n'a pas fait l'objet d'un développement à l'échelle industrielle aussi poussé que la précédente catégorie. En effet, la difficulté majeure avec ce procédé réside dans l'évacuation des calories dégagées lors de la réaction ; évacuation qui, si elle n'est pas suffisante, peut causer un emballement de la réaction mais aussi une baisse des performances du procédé et une dégradation du catalyseur employé.

Une solution préconisée pour l'évacuation de la chaleur de réaction a été de recycler une fraction du mélange réactionnel, refroidi au préalable.

Ainsi, dans la demande de brevet français FR 2126832, on a proposé d'effectuer l'hydrogénation de composés nitrés aromatiques dans un réacteur de forme tubulaire, à lit fixe de type ruisselant (c'est-à-dire injection des réactifs à co-courant du haut vers le bas du réacteur) dans lequel le flux d'alimentation comprend une partie du mélange réactionnel recyclé provenant dudit réacteur. Cependant, les dimensions du réacteur sont telles que l'on ne peut pas considérer ce réacteur comme fonctionnant en adiabatique car 60 à 70 % des échanges thermiques se font par des pertes au niveau des parois dudit réacteur. Par conséquent, une extrapolation à l'échelle industrielle de ce procédé conduirait à avoir des réacteurs dont la hauteur serait considérablement plus grande que la section, de manière à conserver un échange thermique au niveau des parois raisonnable. Toutefois, de tels réacteurs nécessiteraient l'utilisation de quantités de catalyseur très importantes, ce qui diminuerait l'un des intérêts du procédé à lit fixe par rapport aux suspensions de catalyseurs. Une autre possibilité pour l'exploitation à l'échelle industrielle de ce type de procédé, serait d'augmenter les échanges thermiques avec le flux réactionnel recyclé. En d'autres termes, cela consisterait à augmenter de manière considérable le taux de recyclage du milieu réactionnel. Dans ce cas, la productivité d'un tel procédé serait beaucoup trop faible, du fait de la dilution importante requise des composés à nitrer et de la nécessité de convertir totalement ces composés.

Dans la demande de brevet GB 1452466, qui a plus particulièrement pour objet l'hydrogénation du nitrobenzène en aniline, le nitrobenzène est introduit sous forme vaporisée dans le réacteur, où il est mis en contact avec l'hydrogène. Cette opération est effectuée dans un réacteur tubulaire, dont la première comprend un catalyseur en lit fixe et un système de refroidissement, donc ne fonctionnant pas en adiabatique ; et la seconde comprend un catalyseur en lit fixe, fonctionnant en adiabatique. Ce document ne préconise pas une réaction conduite dans un système totalement adiabatique.

La publication "Adiabatic process of catalytic reduction of nitrobenzene to aniline" (IZV. KHIM., vol. 16, no. 1-2, 1983, pp. 38-43) concerne l'hydrogénation de nitrobenzène dans un réacteur adiabatique, utilisant des effluents gazeux comprenant de l'hydrogène.

Comme on peut le constater, il n'y a pas à l'heure actuelle de procédés d'hydrogénation en lit fixe de composés nitrés aromatiques exploitables industriellement de manière satisfaisante.

La présente invention a donc pour objet de proposer un tel type de procédé ne présentant pas les inconvénients précités des réactions d'hydrogénation mettant en oeuvre des catalyseurs en lit fixe.

Ainsi, le procédé selon l'invention présente tous les avantages des procédés mettant en oeuvre des lits fixes comparés aux procédés en suspension agitée de catalyseur.

Notamment, le procédé selon l'invention est de mise en oeuvre facile avec un investissement amoindri. En effet, il n'est plus nécessaire de prévoir des systèmes complexes de séparation du catalyseur une fois la réaction terminée, ni même de prévoir des systèmes d'agitation, avec tous les problèmes d'étanchéité que cela comporte.

De plus, le procédé selon l'invention permet de résoudre les difficultés liées à la conduite de la réaction en lit fixe, c'est-à-dire l'exothermie de la réaction.

Enfin, les performances du procédé selon l'invention, en terme de productivité et de sélectivité, sont tout à fait comparables, en terme de productivité et de sélectivité, à celles obtenues par les procédés utilisant des suspensions de catalyseur agité.

Ces buts et d'autres sont atteints par la présente invention qui a donc pour objet un procédé d'hydrogénation de composés nitrés aromatiques dans lequel on fait réagir au moins un composé nitré aromatique avec de l'hydrogène dans deux réacteurs catalytiques à lit fixe en série fonctionnant en adiabatique ; procédé dans lequel une partie du mélange réactionnel sortant du premier réacteur est recyclée à ce dernier, l'autre partie étant introduite dans le second réacteur.

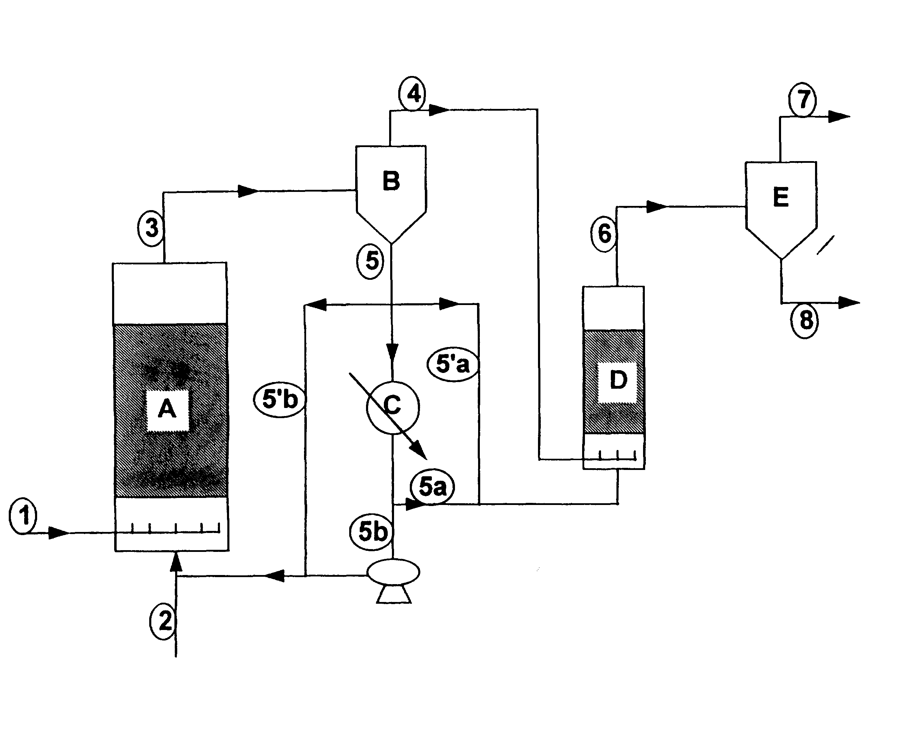

Mais d'autres caractéristiques et avantages de la présente invention seront apparentes à la lecture de la description, de la figure et des exemples qui vont suivre.

Pour des raisons de commodité, les appareillages et leur enchaînement vont tout d'abord être décrits.

Comme cela a été indiqué auparavant, le procédé selon l'invention est réalisé dans un ensemble de deux réacteurs en série.

L'alimentation de chacun des réacteurs a lieu de préférence à co-courant de gaz et de liquide. Il est possible d'injecter les réactifs et/ou le mélange réactionnel par le haut ou par le bas du réacteur.

Dans le premier cas, le procédé est en lit catalytique dit "ruisselant" et la phase continue dans le réacteur est une phase gazeuse.

Selon un mode de réalisation particulièrement avantageux et préféré de l'invention, on alimente chacun des réacteurs avec de rhydrogène, au moins un composé nitré aromatique et/ou le mélange réactionnel recyclé ou non, à co-courant, de manière à ce que la phase continue dans lesdits réacteurs soit liquide.

Un tel résultat est obtenu en alimentant les réacteurs du bas vers le haut. De cette façon, le lit de catalyseur est immergé dans les réactifs et/ou le mélange réactionnel.

Ce mode d'alimentation est particulièrement approprié car il augmente l'efficacité des contacts gaz-liquide et liquide-solide. Par ailleurs, le fait que le catalyseur soit continuellement immergé diminue les risques de dégradation de ce dernier, dus à des surchauffes locales.

On ne sortirait pas du cadre de la présente invention en mettant en oeuvre deux réacteurs alimentés différemment. Cependant, on préfère utiliser deux réacteurs alimentés par le bas.

L'une des caractéristiques essentielles de l'invention réside dans le fait qu'une partie du mélange réactionnel issu du premier réacteur est recyclée dans le flux d'alimentation de ce dernier, l'autre partie étant introduite dans le second réacteur.

Plus particulièrement, le rapport du débit d'alimentation du premier réacteur en composés nitrés aromatiques au débit de mélange réactionnel recyclé dans le premier réacteur est tel que la concentration en composés nitrés soit comprise entre 0,1 et 10 % en poids dans le premier réacteur.

Selon un mode de réalisation particulier de l'invention, le rapport du débit d'alimentation du premier réacteur en composés nitrés aromatiques au débit de mélange réactionnel recyclé dans le premier réacteur est compris entre 5 et 1000.

La température dans le pied du premier réacteur est de l'ordre de 50 à 200 °C et plus particulièrement de l'ordre de 80 à 170 °C.

En sortie du premier réacteur, l'élévation de température, c'est-à-dire la différence de température de sortie du mélange réactionnel et celle d'entrée des composés nitrés et/ou du mélange réactionnel recyclé est inférieure à 120°C.

Le mélange sortant du premier réacteur comprend des composés nitrés n'ayant pas réagi, les composés aromatiques hydrogénés correspondants, de l'eau ainsi que de l'hydrogène.

Selon une variante de l'invention, le mélange réactionnel issu du premier réacteur est introduit dans un séparateur gaz-liquide.

La fraction gazeuse résultante est alimentée au second réacteur tandis que la fraction liquide est séparée en deux parties dont l'une est recyclée dans le flux d'alimentation du premier réacteur.

Cette opération de séparation peut être réalisée dans tout type d'appareillage conventionnel tel que des réservoirs de forme cylindrique, des cyclones.

Préalablement au recyclage dans le premier réacteur, le mélange réactionnel est refroidi pour avoir la température souhaitée après mélange avec les réactifs.

De préférence le mélange réactionnel sortant du premier réacteur est séparé des gaz qu'il contient, préalablement au recyclage à ce dernier et à l'introduction dans le second réacteur.

Par ailleurs, selon un mode de réalisation particulier de l'invention, le mélange réactionnel introduit dans l'échangeur précité est un mélange réactionnel débarrassé de la majeure partie des gaz qu'il contient.

Les réacteurs dans lesquels sont mis en oeuvre le procédé sont de préférence des tubes de forme cylindrique munis des moyens classiques pour retenir le lit fixe de catalyseur et pour distribuer les flux liquide et gazeux.

Comme on peut le constater, les réacteurs employés sont d'une technologie simple et peu coûteuse, ce qui représente un avantage par rapport aux réacteurs avec suspension de catalyseur agité.

Le comportement du premier réacteur, du fait du taux de recyclage important, est du type agité.

En ce qui concerne le second réacteur, on met en oeuvre un réacteur de type piston. L'association des deux réacteurs est avantageuse car elle permet de minimiser la quantité globale de catalyseur et donc la taille des réacteurs.

Le dimensionnement de ces appareillages peut être effectué de manière classique par l'homme du métier en fonction du type de réacteur (agité, piston), de la conversion souhaitée et du débit de composés nitrés à hydrogéner.

La majeure partie de la réaction d'hydrogénation est réalisée dans le premier réacteur. En effet, on atteint une conversion supérieure ou égale à 90 % en composés nitrés aromatiques hydrogénés par rapport aux composés nitrés aromatiques alimentés dans la boucle de recyclage.

Le second réacteur correspond à un finisseur grâce auquel on atteint des conversions de 100 %.

Les composés nitrés aromatiques traités selon le procédé de l'invention comprennent au moins un groupement nitro.

Plus particulièrement, les composés nitrés aromatiques traités comprennent au moins deux groupements nitro.

A titre de composés nitrés aromatiques convenant particulièrement à l'invention, on peut citer le nitrobenzène, les mononitrotoluènes, les dinitrobenzènes, les dinitrotoluènes.

Le procédé selon l'invention peut être avantageusement mis en oeuvre avec un mélange des isomères.

Les composés nitrés aromatiques peuvent être mis en oeuvre en présence d'un solvant desdits composés nitrés aromatiques.

Selon une première variante, le solvant employé est choisi parmi les alcools aliphatiques ou les éthers cyniques, seuls ou en mélange

Plus particulièrement, on utilise à titre d'alcool aliphatique le méthanol, l'éthanol, le propanol, l'isopropanol, seuls ou en mélange

A titre d'éther cynique, on peut citer le dioxane, le tétrahydrofuranne, seuls ou en mélange.

Selon cette variante, les composés nitrés aromatiques neufs, préalablement à l'injection dans le premier réacteur, sont solubilisés dans le solvant ou le mélange de solvants précités.

La concentration en composés nitrés dans le solvant ou le mélange de solvants, peut varier dans un large domaine. On utilise cependant des mélanges monophasiques, c'est-à-dire pour lesquels la limite de solubilité du ou des composés nitrés n'est pas atteinte pour le solvant ou le mélange en question. Sans intention de se limiter, la gamme de concentration est inférieure ou égale à 25 % en poids.

Selon une seconde variante préférée de l'invention, le solvant employé est le mélange réactionnel issu du premier réacteur. Si cette variante est retenue, les composés nitrés aromatiques neufs sont alimentés à l'état liquide ou fondu.

Cette variante est intéressante en ce sens où elle ne dilue pas le réactif comme c'est le cas avec l'usage d'un solvant ou d'un mélange de solvants. Cela contribue notamment à conserver une productivité élevée au procédé. Par ailleurs, cette variante s'affranchit de toute étape supplémentaire de séparation des produits du ou des solvants mis en oeuvre lors de la réaction, qui comporte des risques de pertes des produits hydrogénés.

L'hydrogène employé est plus particulièrement de l'hydrogène pur. Par pur, on entend un gaz comprenant au moins 99 % d'hydrogène, et plus particulièrement au moins 99,9 % d'hydrogène. Il est à noter que l'on ne sortirait pas du cadre de la présente invention en mettant en oeuvre la réaction d'hydrogénation avec de l'hydrogène dilué, bien que cela n'apporte pas d'avantages particulier à la réaction.

L'hydrogène est alimenté dans la quantité stoechiométrique. Cependant, de préférence, on emploie l'hydrogène en excès par rapport aux composés nitrés aromatiques.

Plus particulièrement, la teneur en hydrogène est telle que l'excès par rapport à la stoechiométrie est compris entre 5 et 50 % molaire. Il est à noter que le fait de mettre en oeuvre la réaction d'hydrogénation en présence d'un excès d'hydrogène ne pose pas de problème de perte en ce gaz car celui-ci peut être recyclé.

La pression d'hydrogène dans le réacteur varie entre 5 et 150 bar, de préférence entre 10 et 50 bar.

L'alimentation en ce gaz est effectuée par tout moyen connu de l'homme du métier, permettant d'avoir une répartition homogène de ce gaz au sein du réacteur.

La réaction est mise en oeuvre en présence d'un catalyseur d'hydrogénation classique dans le domaine.

Ainsi, on utilise un catalyseur à base d'un métal du groupe VIII de la classification périodique des éléments, supporté ou non.

Parmi les métaux du groupe VIII, on peut citer tout particulièrement le nickel, le platine, le palladium, ces métaux étant utilisés seuls ou en mélange.

Parmi les catalyseurs non supportés, convient notamment le nickel de Raney.

Dans le cas où l'hydrogénation est effectuée en présence d'un catalyseur supporté, le support peut être choisi parmi les matériaux inertes tels que le kieselguhr, le charbon, l'alumine, la silice.

Dans ce cas, la teneur en métal est habituellement comprise entre 0,1 et 10 % en poids, de préférence entre 0,3 et 5 % en poids.

Le catalyseur se présente sous une forme appropriée à une exploitation en lit fixe. Par exemple les billes, les extrudés, les brisures sont des formes possibles.

Généralement, la taille des objets est comprise entre 1 et 5 mm.

La figure représente un mode de réalisation préféré de l'invention, c'est-à-dire dans lequel le lit catalytique est immergé. Les flux ① et ② représentent respectivement l'hydrogène et le composé aromatique nitré, de préférence à l'état fondu. Le flux① est réparti de façon homogène à l'intérieur du réacteur.

Les flux① et ② sont mis en contact du catalyseur dans le réacteur A.

En sortie du réacteur, le flux ③ est introduit dans un séparateur gaz-liquide B duquel est séparé une fraction gazeuse ④ comprenant majoritairement de l'hydrogène, et une fraction liquide⑤.

Selon un premier mode de réalisation, la totalité du flux ⑤ est dirigée vers l'échangeur C. Cet échangeur a pour objectif de diminuer la température du mélange réactionnel dégazé de manière à obtenir la température souhaitée après mélange avec les composés nitrés du flux ②.

En sortie de l'échangeur C une partie du flux 5a est dirigée vers le réacteur D ; l'autre partie, 5b, est recyclée en pied du réacteur A.

Selon un second mode de réalisation, une partie de ce flux ⑤ est dérivée en amont de l'échangeur C. Ceci permet notamment de diminuer la taille de l'échangeur. Ainsi, selon ce mode de réalisation, on peut dériver une partie du flux, appelée 5'a, et/ou une partie du flux, appelée 5'b, en amont de l'échangeur pour les combiner ensuite avec les flux 5a et 5b respectivement.

La fraction liquide 5a, éventuellement 5'a, et le flux gazeux ④ alimentent le réacteur D de façon à conserver le lit catalytique immergé. Le mélange réactionnel ⑥ est introduit dans un séparateur et la fraction gazeuse ⑦, comprenant majoritairement de l'hydrogène peut être avantageusement recyclée en amont du procédé, par exemple à l'alimentation du réacteur A. La fraction liquide⑧ comprenant l'eau et les composés aromatiques hydrogénés sont traités pour éliminer l'eau.

Les composés hydrogénés peuvent notamment être employés pour la préparation d'isocyanates, intermédiaires des polyuréthannes.

L'exemple suivant illustre l'invention sans la limiter.

### EXEMPLE

La réaction d'hydrogénation est mise en oeuvre dans un appareillage dont l'agencement correspond à celui de la figure.

Le réacteur A correspond à un tube de diamètre de 45 mm et d'une hauteur de 800 mm.

Le réacteur D correspond à un tube de diamètre de 15 mm et d'une hauteur de 500 mm.

Le réacteur A comprend 643 g d'un catalyseur Pd/C comprenant 0,5 % en poids de palladium, sous la forme de brisures d'une taille moyenne de 3 mm environ.

Le réacteur D comprend 50 g du même catalyseur que précédemment.

On introduit du dinitrotoluène industriel comprenant 96 % d'un mélange d'isomères 2.4/2.6 dans un rapport 4/1 et 4 % d'isomères 3.5/3.4/2.5, avec un débit de 245 g/h.

Le débit d'hydrogène est de 580 NI/h.

La pression dans le réacteur est de 25 bar.

La température d'entrée dans ce réacteur est de 80 °C et celle de sortie de 100 °C.

Un débit de 20 l/h de mélange réactionnel dégazé et refroidi pour obtenir une température voisine de 80 °C, est recyclé au réacteur A.

Dans ce réacteur, le taux de conversion du dinitrotoluène alimenté est supérieur à 95 %.

On alimente le réacteur D de telle sorte que le volume dans le premier réacteur reste constant.

La pression du réacteur D est identique et la température d'entrée est de 80 °C.

En sortie de ce second réacteur, la conversion des dinitrotoluènes est totale et la sélectivité supérieure à 98 %.

## Revendications

1. Procédé d'hydrogénation de composés nitrés aromatiques, caractérisé en ce que l'on fait réagir au moins un composé nitré aromatique avec de l'hydrogène dans deux réacteurs catalytiques à lit fixe en série, fonctionnant en adiabatique ; procédé dans lequel une partie du mélange réactionnel sortant du premier réacteur est recyclé à ce dernier, l'autre partie étant introduite dans le second réacteur ; et dans lequel on alimente chacun des réacteurs avec de l'hydrogène, au moins un composé nitré et/ou le mélange réactionnel recyclé ou non, à co-courant, de manière à ce que la phase continue dans lesdits réacteurs soit liquide.

2. Procédé selon la revendication 1, caractérisé en ce que le rapport du débit d'alimentation du premier réacteur en composés nitrés au débit de mélange réactionnel recyclé dans le premier réacteur est tel que la concentration en composés nitrés aromatiques soit comprise entre 0,1 et 10 % en poids dans le premier réacteur.

3. Procédé selon l'une quelconque des revendications précédentes, caractérisé en ce que l'on effectue la réaction de telle sorte que la différence entre la température de sortie du mélange réactionnel et celle d'entrée des composés nitrés et/ou du mélange réactionnel recyclé est inférieure à 120°C.

4. Procédé selon l'une quelconque des revendications précédentes, caractérisé en ce que l'on sépare les gaz du mélange réactionnel sortant du premier réacteur, avant le recyclage à ce dernier, et avant l'introduction dans le second réacteur.

5. Procédé selon l'une quelconque des revendications précédentes, caractérisé en ce que l'on fait réagir des composés nitrés aromatiques comprenant au moins un groupement nitré.

6. Procédé selon l'une quelconque des revendications précédentes, caractérisé en ce que l'on effectue la réaction en présence d'un solvant desdits composés nitrés aromatiques.

7. Procédé selon la revendication précédente, caractérisé en ce que l'on effectue la réaction en présence d'un solvant choisi parmi les alcools aliphatiques ou les éthers cycliques, seuls ou en mélange.

8. Procédé selon la revendication précédente, caractérisé en ce que l'on effectue la réaction en présence d'un solvant qui est le mélange réactionnel.

9. Procédé selon l'une quelconque des revendications précédentes, caractérisé en ce que l'on effectue la réaction en présence d'un excès d'hydrogène par rapport à la stoechiométrie de 5 à 50 % molaire.

10. Procédé selon l'une quelconque des revendications précédentes, caractérisé en ce que l'on effectue la réaction en présence d'un catalyseur à base d'au moins un métal du groupe VIII, supporté ou non.

## Patentansprüche

1. Verfahren zur Hydrierung von aromatischen Nitroverbindungen, dadurch gekennzeichnet, daß man mindestens eine aromatische Nitroverbindung mit Wasserstoff in zwei hintereinander angeordneten katalytischen Festbettreaktoren, die adiabatisch arbeiten, reagieren läßt, in welchem Verfahren ein Teil der Reaktionsmischung, die aus dem ersten Reaktor austritt, zu diesem letzteren zurückgeführt wird, wobei der andere Teil in den zweiten Reaktor eingespeist wird, und in welchem man in jeden der Reaktoren Wasserstoff, mindestens eine Nitroverbindung und/oder die zurückgeführte oder nicht zurückgeführte Reaktionsmischung im Gleichstrom so einspeist, daß die kontinuierliche Phase in diesen Reaktoren flüssig ist.

2. Verfahren nach Anspruch 1, dadurch gekennzeichnet, daß das Verhältnis der Einspeismenge an Nitroverbindungen in den ersten Reaktor zu der Menge von in den ersten Reaktor zurückgeführter Reaktionsmischung so ist, daß die Konzentration an aromatischen Nitroverbindungen in dem ersten Reaktor zwischen 0,1 und 10 Gew.-% beträgt.

3. Verfahren nach einem der vorangegangenen Ansprüche, dadurch gekennzeichnet, daß man die Umsetzung so ausgeführt, daß der Unterschied zwischen der Austrittstemperatur der Reaktionsmischung und der Eintrittstemperatur der Nitroverbindungen und/oder der zurückgeführten Reaktonsmischung unter 120°C beträgt.

4. Verfahren nach einem der vorangegangenen Ansprüche, dadurch gekennzeichnet, daß man die Gase von der aus dem ersten Reaktor austretenden Reaktionsmischung vor dem Zurückführen zu diesem letzteren und vor dem Einspeisen in den zweiten Reaktor abtrennt.

5. Verfahren nach einem der vorangegangenen Ansprüche, dadurch gekennzeichnet, daß man aromatische Nitroverbindungen, die mindestens eine Nitrogruppe enthalten, reagieren läßt.

6. Verfahren nach einem der vorangegangenen Ansprüche, dadurch gekennzeichnet, daß man die Umsetzung in Gegenwart eines Lösemittels für die aromatischen Nitroverbindungen ausführt.

7. Verfahren nach dem vorangegangenen Anspruch, dadurch gekennzeichnet, daß man die Umsetzung in Gegenwart eines Lösemittels, ausgewählt unter den aliphatischen Alkoholen oder den cyclischen Ethern, allein oder in einer Mischung, ausführt.

8. Verfahren nach dem vorangegangenen Anspruch, dadurch gekennzeichnet, daß man die Umsetzung in Gegenwart eines Lösemittels, das die Reaktionsmischung ist, ausführt.

9. Verfahren nach einem der vorangegangenen Ansprüche, dadurch gekennzeichnet, daß man die Umsetzung in Gegenwart eines Überschusses von Wasserstoff von 5 bis 50 mol-% bezogen auf die Stöchiometrie ausführt.

10. Verfahren nach einem der vorangegangenen Ansprüche, dadurch gekennzeichnet, daß man die Umsetzung in Gegenwart eines Katalysators auf Grundlage von mindestens einem Metall der Gruppe VIII, der auf einen Träger aufgebracht ist oder nicht, ausführt.

## Claims

1. Process for hydrogenating aromatic nitro compounds, characterized in that at least one aromatic nitro compound is reacted with hydrogen in two fixedbed catalytic reactors in series operating adiabatically; in this process, some of the reaction mixture leaving the first reactor is recycled into this reactor, the other part being introduced into the second reactor; and in which each of the reactors is supplied with hydrogen, at least one nitro compound and/or the recycled or non-recycled reaction mixture, co-currentwise, such that the continuous phase in the said reactors is liquid.

2. Process according to claim 1, characterized in that the ratio of the flow rate of supply of the first reactor with nitro compounds to the flow rate of reaction mixture recycled into the first reactor is such that the concentration of aromatic nitro compounds is between 0.1 and 10% by weight in the first reactor.

3. Process according to any one of the preceding claims, characterized in that the reaction is carried out such that the difference between the outlet temperature of the reaction mixture and the inlet temperature of the nitro compounds and/or of the recycled reaction mixture is less than 120°C.

4. Process according to any one of the preceding claims, characterized in that the gases are separated from the reaction mixture leaving the first reactor, before recycling into this reactor and before introduction into the second reactor.

5. Process according to any one of the preceding claims, characterized in that aromatic nitro compounds comprising at least one nitro group are reacted.

6. Process according to any one of the preceding claims, characterized in that the reaction is carried out in the presence of a solvent for the said aromatic nitro compounds.

7. Process according to the preceding claim, characterized in that the reaction is carried out in the presence of a solvent chosen from aliphatic alcohols and cyclic ethers, alone or as a mixture.

8. Process according to the preceding claim, characterized in that the reaction is carried out in the presence of a solvent which is the reaction mixture.

9. Process according to any one of the preceding claims, characterized in that the reaction is carried out in the presence of an excess of hydrogen, relative to the stoichiometry, of 5 to 50 mol%.

10. Process according to any one of the preceding claims, characterized in that the reaction is carried out in the presence of a catalyst based on at least one metal from group VIII, which may or may not be supported.
